# EUROPÄISCHE PATENTSCHRIFT

(11) **EP 0 408 542 B1**
(45) Veröffentlichungstag und Bekanntmachung des Hinweises auf die Patenterteilung: **20.09.1995**
(21) Anmeldenummer: 90890206.7
(22) Anmeldetag: 09.07.1990
(51) Int. Cl.: G01N 33/53, G01N 33/543

(54) **Verfahren zur Bestimmung von Antigenen und/oder Antikörpern in menschlichen Körperflüssigkeiten sowie Set zur Durchführung des Verfahrens**
A method of determining antigens and/or antibodies in human body liquids, and a test kit for carrying out this method
Procédé pour la détermination d'antigènes et/ou d'anticorps dans des liquides corporelles humaines et kit pour la mise en oeuvre de ce procédé

(30) Priorität: 13.07.1989 AT 1699/89
(43) Veröffentlichungstag der Anmeldung: 16.01.1991
(73) Patentinhaber: IMMUNO Aktiengesellschaft, A-1221 Wien (AT)
(72) Erfinder: Aicher, Helmut, Dr., A-2238 Obersiebenbrunn (AT); Molinari, Ewald, Dr., A-2340 Mödling (AT)
(74) Vertreter: Wolfram, Gustav, Dipl.-Ing.

(56) Entgegenhaltungen:
- EP-A- 0 042 755
- WO-A-83/03677
- GB-A- 2 051 357
- BIOTECHNIQUES, Band 6, Nr. 4, 01 April 1988, Natick, MA (US); T.OBATA et al., Seiten 299-303

## Beschreibung

Die Erfindung betrifft ein Verfahren zur Bestimmung von Antigenen und/oder Antikörpern in menschlichen Körperflüssigkeiten, durch Bildung eines Antigen/Antikörper-Komplexes, wobei die auf Antigen- bzw. Antikörpergehalt zu prüfende Probe mit einem komplexbildenden Antikörper bzw. Antigen, welcher(s) auf einem Träger in festem Zustand adsorbiert ist, in Kontakt gebracht wird, sowie ein Set zur Durchführung des Verfahrens.

Aus der DE-C - 24 24 465 ist ein Verfahren zur gleichzeitigen Bestimmung von Hepatitis B-Antigen und dessen Antikörper in Seren bekannt. Das Bestimmungsprinzip ist eine Inhibitionsreaktion, die naturgemäß die Empfindlichkeit des Systems stark herabsetzt.

Die DE-A - 30 21 891 beschreibt ein Verfahren zum gleichzeitigen Nachweis von verschiedenen Hepatitis-Indikatoren, u.zw. Hepatitis B-Oberflächenantigen und einem Antikörper gegen Hepatitis B-Kernantigen. Der Nachteil dieses Verfahrens besteht darin, daß die beiden zu bestimmenden Stoffe mit zwei gänzlich verschiedenen Reaktionstypen gesucht werden müssen, wobei der eine Reaktionspartner mit einem radioaktiven Isotop markiert werden muß, während der andere enzymatisch bestimmt wird.

Ganz allgemein muß bei der Bestimmung von mindestens zwei Immunreaktanten auf die strenge Einhaltung einer ganz bestimmten Abfolge von Verfahrensschritten geachtet werden, was naturgemäß die Gefahr von Fehlbestimmungen sehr erhöht. Die Fehlerquelle wird dabei umso größer, je mehr Immunreaktanten bestimmt werden sollen. Dazu kommt noch, daß bei der Bestimmung einer Mehrzahl von Stoffen die Probe entsprechend oft pipettiert werden muß, wodurch die Genauigkeit der Einzelwerte oft nachteilig beeinflußt wird.

Die Erfindung setzt sich zum Ziel, ein einfach durchzuführendens Verfahren zur Bestimmung einer Mehrzahl von Antigenen und/oder Antikörpern in Proben menschlicher Körperflüssigkeiten zur Verfügung zu stellen, das die oben genannten Nachteile nicht aufweist und ohne radioaktive Markierung durchgeführt werden kann.

Dieses Ziel wird erfindungsgemäß dadurch erreicht, daß zur gleichzeitigen Bestimmung einer Mehrzahl von in der zu untersuchenden Probe enthaltenden Antigenen Agₐ₁, Agₐ₂,...Ag_{b1}, Ag_{b2},... die Probe in eine Mikrotiterplatte eingebracht wird, deren Näpfchen mit Antikörpern Ak_{a1,} Ak_{a2,}... beschichtet sind, die gegen einen Teil der gesuchten Antigene Agₐ₁, Agₐ₂,... gerichtet sind, und daß eine Mehrzahl fester Träger, vorzugsweise in Form eines Kammes, die mit Antikörpern Ak_{b1}, Ak_{b2},... beschichtet sind die gegen einen anderen Teil der gesuchten Antigene Ag_{b1}, Ag_{b2},... gerichtet sind, mit der Probe in Kontakt gebracht wird; sodann - nach erfolgter Komplexierung - die Näpfchen und die festen Träger von nicht adsorbierten Proteinen freigewaschen werden, die in den Näpfchen der Mikrotiterplatte und/oder an den festen Trägern adsorbierten Komplexe Akₐ₁-Agₐ₁,...und/oder Ak_{b1}-Ag_{b1},... mit einem Gemisch von markierten Antikörperenzymen Akₐ₁E*,...Ak_{b1}E*,..., die gegen die gesuchten Antigene Agₐ₁, Agₐ₂,...Ag_{b1}, Ag_{b2},... gerichtet sind, inkubiert werden, die festen Träger nach der Inkubation mit dem Gemisch von markierten Antikörperenzymen in eine zweite Mikrotiterplatte eingesetzt werden und die Aktivität der an die Komplexe gebundenen Enzyme (Akₐ₁-Agₐ₁,-Akₐ₁E*...Ak_{b1}-Ag_{b1}-Ak_{b1}E*,...) nach Umsetzung mit einem färbendem Substrat photometrisch bestimmt wird.

Zur gleichzeitigen Bestimmung einer Mehrzahl von in der zu untersuchenden Probe enthaltenen Antikörpern Akₐ₁, Akₐ₂,...Ak_{b1}, Ak_{b2},... wird die Probe in eine Mikrotiterplatte eingebracht, deren Näpfchen mit Antigenen Agₐ₁, Agₐ₂,... beschichtet sind, die mit einem Teil der gesuchten Antikörper Akₐ₁, Akₐ₂,... korrespondieren, und wird eine Mehrzahl fester Träger, vorzugsweise in Form eines Kammes, die mit Antigenen Ag_{b1}, Ag_{b2},... beschichtet sind, die mit einem anderen Teil der gesuchten Antikörper Ak_{b1}, Ak_{b2},... korrespondieren, mit der Probe in Kontakt gebracht; sodann werden - nach erfolgter Komplexierung - die Näpfchen und die festen Träger von nicht adsorbierten Proteinen freigewaschen, die in den Näpfchen der Mikrotiterplatte und/oder an den festen Trägern adsorbierten Komplexe Agₐ₁-Akₐ₁,...und/oder Ag_{b1} -Ak_{b1},... mit einem Gemisch von markierten Antigenenzymen Agₐ₁E*,...Ag_{b1}E*,..., die mit den gesuchten Antikörper Akₐ₁, Akₐ₂,...Ak_{b1}, Ak_{b2},... korrespondieren, inkubiert werden, die festen Träger nach der Inkubation mit dem Gemisch von markierten Antigenenzymen in eine zweite Mikrotiterplatte eingesetzt werden und die Aktivität der an die Komplexe gebundenen Enzyme Agₐ₁ -Akₐ₁ -Agₐ₁E*,...Ab_{b1}-Ak_{b1}-Ag_{b1}E*,... nach Umsetzung mit einem färbendem Substrat photometrisch bestimmt.

Das erfindungsgemäße Verfahren dient auch zur gleichzeitigen Bestimmung mindestens eines Antikörpers (Akₐ₁,...) und mindestens eines Antigens (Ag_{b1},...) in menschlichen Körperflüssigkeiten durch Bildung von Antikörper/Antigen- bzw. Antigen-Antikörper-Komplexen, wobei die auf Antikörper- bzw. Antigengehalt zu prüfende Probe mit einem komplexbildenden Antigen bzw. Antikörper, welcher auf einem Träger in festem Zustand adsorbiert ist, in Kontakt gebracht wird, wobei das Verfahren dadurch gekennzeichnet ist, daß die Probe in eine Mikrotiterplatte eingebracht wird, und daß eine Mehrzahl fester Träger, vorzugsweise in Form eines Kammes mit der Probe in Kontakt gebracht wird, wobei die Näpfchen der Mikrotiterplatte und die festen Träger mit dem(den) zu dem(den) gesuchten Antikörper(n) Akₐ₁,... bzw. Antigen(en) Ag_{b1},... korrespondierendem(n) Antigen(en) Agₐ₁,... bzw. Antikörper(n) Ak_{b1},... beschichtet ist(sind); sodann - nach erfolgter Komplexierung - die Näpfchen und die festen Träger von nicht adsorbierten Proteinen freigewaschen werden, die in den Näpfchen der Mikrotiterplatte und/oder an den festen Trägern adsorbierten Komplexe Agₐ₁ -Akₐ₁,... bzw. Ak_{b1} -Ag_{b1},... mit einem Gemisch von markierten Antigenenzymen Agₐ₁E*,... bzw. Antikörperenzymen Ak_{b1}E*,..., die mit dem(den) gesuchten Antikörper(n) (Akₐ₁,...) bzw. Antigen(en) (Ag_{b1},...) korrespondieren, inkubiert werden, die festen Träger nach der Inkubation mit dem Gemisch von markierten Antigen- bzw. Antikörperenzymen in eine zweite Mikrotiterplatte eingesetzt werden und die Aktivität der an die Komplexe gebundenen Enzyme Agₐ₁ -Akₐ₁ -Agₐ₁E*,... bzw. Ak_{b1},-Ag_{b1}-Ak_{b1}E*,... nach Umsetzung mit einem farbendem Substrat photometrisch bestimmt wird.

Mit dem erfindungsgemäßen Verfahren kann somit eine Mehrzahl von Antikörpern und/oder Antigenen in einer Probe bestimmt werden, wobei die Probe nur ein einziges Mal pipettiert zu werden braucht. Die Gefahr von Pipettierfehlern im täglichen Routinebetrieb wird beim erfindungsgemäßen Verfahren somit auf ein Minimum reduziert.

Die Erfindung betrifft auch ein Set zur Durchführung des Verfahrens, mit einer Mikrotiterplatte und einem in die Näpfchen der Mikrotiterplatte passenden festen Träger, insbesondere einem kammartigen Träger, und ist dadurch gekennzeichnet, daß die Näpfchen der Mikrotiterplatte und die festen Träger mit einem oder mehreren Antigen(en) Agₐ₁, Ag_{b1},... bzw. mit einem oder mehreren Antikörper(n) Akₐ₁, Ak_{b1},... beschichtet ist (sind), welche mit dem (den) gesuchten Antigen(en) und/oder Antikörper(n) Komplexe bilden.

Ein Set, bestehend aus einer Mikrotiterplatte und einem kammartigen Träger zur simultanen radioimmunchemischen Bestimmung eines Antigens und eines Antikörpers ist aus der AT-B-343 822 bekannt.

Mit den nachfolgenden Beispielen wird die Erfindung noch näher erläutert.

### Beispiel 1:

### Gleichzeitige Bestimmung von Apo-Lipoprotein-Al (antiatherosklerotisches HD-Lipoprotein) und Apo-Protein-B (atherosklerotisches LD-Lipoprotein) in biologischen FLüssigkeiten

Näpfchen einer Mikrotiterplatte wurden mit 200 µl (pro Näpfchen) einer Lösung eines affinitätsgereinigten Schafsantikörpers gegen Apo-A in einem 0,1 m PBS-Puffer (pH: 7,4) mit einer Antikörperkonzentration von 1 µg/ml 12 Stunden bei Zimmertemperatur behandelt und mit dem Antikörper beschichtet. Danach wurden die Näpfchen mindestens dreimal mit 250 µl PBS-Puffer, enthaltend 0,05% Tween™ 20 gewaschen. Allfällige nicht besetzte Bindungsstellen an der Polystyrolwand der Näpfchen wurden durch Zugabe einer 0,2 %, w/v Albuminlösung in PBS-Puffer zwei Stunden bei Zimmertemperatur abgesättigt, worauf die Platten neuerlich gewaschen und einer gründlichen Trocknung unter Vakuum unterzogen wurden. Die Aufbewahrung der Platten erfolgte unter Silikagel.

In ähnlicher Weise wurden die Plättchen des Testkammes mit einem affinitätsgereinigten Antikörper gegen Apo-B beschichtet.

Die zu untersuchenden Proben und Standards wurden in PBS-Tween™-Puffer 1 : 500 vorverdünnt und in der Mikrotiterplatte unter Zugabe des Testkammes zwei Stunden bei Zimmertemperatur inkubiert. Das Probevolumen betrug 200 µl. Danach wurden die Näpfchen der Mikrotiterplatte sowie die Testkämme gründlich in PBS-Tween-Puffer gewaschen und 200 µl eines Gemisches aus mit Meerettichperoxidase markierten gereinigten Antikörpern gegen Apo-Al und Apo-B in die Näpfchen pipettiert. Die Testkämme wurden eingesetzt und das Set eine Stunde bei Zimmertemperatur inkubiert. Nach einem neuerlichen Waschschritt wurden in die mit anti-apo-Al beschichtete Mikrotiterplatte sowie in eine zweite Mikrotiterplatte, die unbeschichtet war, jeweils 200 µl Tetramethylbenzidin enthaltendes Substrat pipettiert.

Die Testkämme wurden in die zweite Mikrotiterplatte eingesetzt und beide Platten 30 min bei Zimmertemperatur inkubiert. Danach wurde die Enzymsubstratreaktion in beiden Platten durch Zugabe von 50 µl 4 n Schwefelsäure gestoppt. Mit Hilfe eines Photometers wurden die Extinktionen der einzelnen Proben in beiden Platten bei 450 nm gemessen und die Konzentrationen an Apo-Al und Apo-B in den Proben durch Vergleich mit den mitgeführten Standards ermittelt.

Nach dem erfindungsgemäßen Verfahren kann auch der Quotient der Konzentrationen der korrespondierenden Proben gebildet werden, was für die medizinische Diagnose bzw. Prognose von Bedeutung ist.

### Beispiel 2:

### Gleichzeitige Bestimmung von IgM-Antikörpern gegen FSME, IgG-Antikörpern gegen FSME und Antikörpern gegen Borrelia-Burgdorferie

Näpfchen einer Mikrotiterplatte wurden analog Beispiel 1 mit einer gereinigten Virus-Suspension von FSME-Antigen und die Plättchen der Kämme mit einer Suspension von Borrelia-Burgdorferie-Antigen beschichtet. In die Mikrotiterplatte wurden 200 µl einer 1 : 50-Verdünnung der Proben vorgelegt, die Testkämme eingesetzt und das Set zwei Stunden bei 45°C inkubiert. Die Näpfchen der Mikrotiterplatte und die Testkämme wurden gewaschen, in die beschichtete Mikrotiterplatte ein Gemisch von Anti-human IgG, markiert mit alkalischer Phosphatase (Enzym 1), und FSME-Antigen, markiert mit Meerettichperoxidase (Enzym 2), pipettiert.

In eine zweite, unbeschichtete Mikrotiterplatte wurde ebenfalls anti-Human IgG, mit Enzym 1 markiert, vorgelegt, und die Testkämme wurden eingesetzt. Beide Platten wurden eine Stunde bei 45°C inkubiert, die Platten sowie die Testkämme nochmals gewaschen, und in die beschichtete Mikrotiterplatte wurde ein Gemisch von o-Phenylendiamin und p-Nitrophenylphosphat (PNPP) in geeigneter gepufferter Lösung einpipettiert.

In die zweite Platte wurde PNPP vorgelegt und die Testkämme wurden eingesetzt. Beide Platten wurden 30 min bei Raumtemperatur inkubiert und danach die Testkämme verworfen. Die Reaktion in Platte 2 wurde durch Zugabe von 50 µl 4 n Schwefelsäure gestoppt. Bei Platte 1 wurden in einem geeigneten Photometer die Extinktionen in den einzelnen Näpfchen gemessen (Messung der Enzymsubstratextinktion des Enzyms 1 bei 405 nm; danach wurden 50 µl 4 n Schwefelsäure zugegeben und die Enzymsubstrat-Extinktionen des Enzyms 2 bei 492 nm gemessen).

Die Extinktionen von Enzym-Substrat Nr. 1 entsprechen dem Anti-FSME-IgG Gehalt der Probe, die Extinktionen von Enzym-Substrat Nr. 2 entsprechen dem Anti-FSME-IgM Gehalt der Probe.

In einem nächsten Schritt erfolgte die Messung der Extinktionen in Platte 2 bei der für das Enzym 1 geeigneten Wellenlänge (405 nm). In Platte Nr. 2 entsprechen die gemessenen Extinktionen dem Gehalt an IgG-Antikörpern gegen Borrelia-Burgdorferie.

Somit ist es möglich, in einem einzigen Testansatz einen Überblick über allfällige Infektionen durch FSME und/oder Borrelia-Burgdorferie zu erhalten.

## Patentansprüche

1. Verfahren zur Bestimmung von Antigenen in menschlichen Körperflüssigkeiten durch Bildung eines Antigen/Antikörper-Komplexes, wobei die auf Antigengehalt zu prüfende Probe mit einem komplexbildenden Antikörper, welcher auf einem Träger in festem Zustand adsorbiert ist, in Kontakt gebracht wird, dadurch gekennzeichnet, daß zur gleichzeitigen Bestimmung einer Mehrzahl von in der zu untersuchenden Probe enthaltenden Antigenen (Agₐ₁, Agₐ₂,...Ag_{b1}, Ag_{b2},...) die Probe in eine Mikrotiterplatte eingebracht wird, deren Näpfchen mit Antikörpern (Akₐ₁, Akₐ₂,...) beschichtet sind, die gegen einen Teil der gesuchten Antigene (Agₐ₁, Agₐ₂,...) gerichtet sind, und daß eine Mehrzahl fester Träger, vorzugsweise in Form eines Kammes, die mit Antikörpern (Ak_{b1}, Ak_{b2},...) beschichtet sind, die gegen einen anderen Teil der gesuchten Antigene (Ag_{b1}, Ag_{b2},...) gerichtet sind, mit der Probe in Kontakt gebracht wird; sodann - nach erfolgter Komplexierung - die Näpfchen und die festen Träger von nicht adsorbierten Proteinen freigewaschen werden, die in den Näpfchen der Mikrotiterplatte und/oder an den festen Trägern adsorbierten Komplexe (Akₐ₁ -Agₐ₁,...und/oder Ak_{b1} -Ag_{b1},...) mit einem Gemisch von markierten Antikörperenzymen (Akₐ₁E*,...Ak_{b1}E*,...), die gegen die gesuchten Antigene (Agₐ₁, Agₐ₂,...Ag_{b1}, Ag_{b2},...) gerichtet sind, inkubiert werden, die festen Träger nach der Inkubation mit dem Gemisch von markierten Antikörperenzymen in eine zweite Mikrotiterplatte eingesetzt werden und die Aktivität der an die Komplexe gebundenen Enzyme (Akₐ₁ -Agₐ₁ -Akₐ₁E*...Ak_{b1}-Ag_{b1}-Ak_{b1}E*,...) nach Umsetzung mit einem färbendem Substrat photometrisch bestimmt wird.

2. Verfahren zur Bestimmung von Antikörpern in menschlichen Körperflüssigkeiten durch Bildung eines Antikörper/Antigen-Komplexes, wobei die auf Antikörpergehalt zu prüfende Probe mit einem komplexbildenden Antigen, welcher auf einem Träger in festem Zustand adsorbiert ist, in Kontakt gebracht wird, dadurch gekennzeichnet, daß zur gleichzeitigen Bestimmung einer Mehrzahl von in der zu untersuchenden Probe enthaltenden Antikörpern (Akₐ₁, Akₐ₂,...Ak_{b1}, Ak_{b2},...) die Probe in eine Mikrotiterplatte eingebracht wird, deren Näpfchen mit Antigenen (Agₐ₁, Agₐ₂,...) beschichtet sind, die mit einem Teil der gesuchten Antikörper (Akₐ₁, Akₐ₂,...) korrespondieren, und daß eine Mehrzahl fester Träger, vorzugsweise in Form eines Kammes, die mit Antigenen (Ag_{b1}, Ag_{b2},...) beschichtet sind, die mit einem anderen Teil der gesuchten Antikörper (Ak_{b1}, Ak_{b2},...) korrespondieren, mit der Probe in Kontakt gebracht wird; sodann - nach erfolgter Komplexierung - die Näpfchen und die festen Träger von nicht adsorbierten Proteinen freigewaschen werden, die in den Näpfchen der Mikrotiterplatte und/oder an den festen Trägern adsorbierten Komplexe (Agₐ₁,-Akₐ₁,... und/oder Ag_{b1}-Ak_{b1},...) mit einem Gemisch von markierten Antigenenzymen (Agₐ₁E*,...Ag_{b1}E*,...) die mit den gesuchten Antikörper (Akₐ₁, Akₐ₂,...Ak_{b1}, Ak_{b2},...) korrespondieren, inkubiert werden, die festen Träger nach der Inkubation mit dem Gemisch von markierten Antigenenzymen in eine zweite Mikrotiterplatte eingesetzt werden und die Aktivität der an die Komplexe gebundenen Enzyme (Agₐ₁-Akₐ₁-Agₐ₁E*,...Ag_{b1}-Ak_{b1}-Ag_{b1}E*,...) nach Umsetzung mit einem färbendem Substrat photometrisch bestimmt wird.

3. Verfahren zur gleichzeitigen Bestimmung mindestens eines Antikörpers (Akₐ₁,...) und mindestens eines Antigens (Ag_{b1},...) in menschlichen Körperflüssigkeiten durch Bildung von Antikörper/Antigen- bzw. Antigen-Antikörper-Komplexen, wobei die auf Antikörper- bzw. Antigengehalt zu prüfende Probe mit einem komplexbildenden Antigen bzw. Antikörper, welcher auf einem Träger in festem Zustand adsorbiert ist, in Kontakt gebracht wird, dadurch gekennzeichnet, daß die Probe in eine Mikrotiterplatte eingebracht wird, und daß eine Mehrzahl fester Träger, vorzugsweise in Form eines Kammes mit der Probe in Kontakt gebracht wird, wobei die Näpfchen der Mikrotiterplatte und die festen Träger mit dem(den) zu dem(den) gesuchten Antikörper(n) (Akₐ₁,...) bzw. Antigen(en) (Ag_{b1},...) korrespondierendem(n) Antigen(en) (Agₐ₁,...) bzw. Antikörper(n) (Ak_{b1},...) beschichtet ist(sind); sodann - nach erfolgter Komplexierung - die Näpfchen und die festen Träger von nicht adsorbierten Proteinen freigewaschen werden, die in den Näpfchen der Mikrotiterplatte und/oder an den festen Trägern adsorbierten Komplexe (Agₐ₁ -Ak_{a1,},... bzw. Ak_{b1} -Ag_{b1},...) mit einem Gemisch von markierten Antigenenzymen (Agₐ₁E*,...) bzw. Antikörperenzymen (Ak_{b1}E*,...) , die mit dem(den) gesuchten Antikörper(n) (Akₐ₁,...) bzw. Antigen(en) (Ag_{b1},...) korrespondieren, inkubiert werden, die festen Träger nach der Inkubation mit dem Gemisch von markierten Antigen- bzw. Antikörperenzymen in eine zweite Mikrotiterplatte eingesetzt werden und die Aktivität der an die Komplexe gebundenen Enzyme (Agₐ₁ -Akₐ₁ -Agₐ₁E*,... bzw. Ak_{b1} -Ag_{b1} -Ak_{b1}E*,...) nach Umsetzung mit einem färbendem Substrat photometrisch bestimmt wird.

4. Set zur Durchführung des Verfahrens nach einem oder mehreren der Ansprüche 1 bis 3, mit einer Mikrotiterplatte und einem in die Näpfchen der Mikrotiterplatte passenden festen Träger, insbesondere einem kammartigen Träger, dadurch gekennzeichnet, daß die Näpfchen der Mikrotiterplatte und die festen Träger mit einem oder mehreren Antigen(en) (Agₐ₁, Ag_{b1},...) bzw. mit einem oder mehreren Antikörper(n)(Akₐ₁, Ak_{b1},...) beschichtet ist (sind), welche mit dem (den) gesuchten Antigen(en) und/oder Antikörper(n) Komplexe bilden.

## Claims

1. A method of determining antigens in human body liquids by the formation of an antigen/antibody complex, wherein the sample to be examined for its antigen content is contacted with a complexing antibody, which is adsorbed on a solid support, characterised in that for a simultaneous determination of a plurality of antigens (Agₐ₁, Agₐ₂,...Ag_{b1}, Ag_{b2},...) contained in the sample to be assayed, the sample is introduced into a microtiter plate whose wells are coated with antibodies (Akₐ₁, Akₐ₂,...) directed against one portion of the antigens (Agₐ₁, Agₐ₂,...) to be detected, and that a plurality of solid supports, preferably in the form of a comb, which are coated with antibodies (Ak_{b1}, Ak_{b2},...) directed against another portion of the antigens (Ag_{b1}, Ag_{b2},...) to be detected, are contacted with the sample; that, after complexing has been effected, the wells and the solid supports are washed free of non-adsorbed proteins, the complexes (Akₐ₁ -Agₐ₁,... and/or Ak_{b1}-Ag_{b1},...) adsorbed in the wells of the microtiter plate and/or on the solid supports are incubated with a mixture of labelled antibody enzymes (Akₐ₁E*,.. Ak_{b1}E*,...) directed against the antigens (Agₐ₁, Agₐ₂,...Ag_{b1}, Ag_{b2},...) to be detected, the solid supports, after incubation with the mixture of labelled antibody enzymes, are inserted into a second microtiter plate and the activities of the enzymes bound to the complexes (Akₐ₁-Agₐ₁ -Akₐ₁,E*,...Ak_{b1} -Ag_{b1} -Ak_{b1}E*,...) are photometrically determined upon reaction with a staining substrate.

2. A method of determining antibodies in human body liquids by the formation of an antibody/antigen complex, wherein the sample to be assayed for its antibody content is contacted with a complexing antigen which has been adsorbed on a solid support, characterised in that for a simultaneous determination of a plurality of antibodies (Akₐ₁, Akₐ₂,...Ak_{b1}, Ak_{b2},...) contained in the sample to be assayed, the sample is introduced into a microtiter plate whose wells have been coated with the antigens (Agₐ₁, Agₐ₂,...) corresponding to a portion of the antibodies (Akₐ₁, Akₐ₂,...) to be detected, and that a plurality of solid supports, preferably in the form of a comb, which are coated with antigens (Ag_{b1}, Ag_{b2},...) which correspond with another portion of the antibodies (Ak_{b1}, Ak_{b2},...) to be detected, are contacted with the sample; whereupon - after complexing has been effected - the wells and the solid supports are washed free of non-adsorbed proteins, the complexes (Agₐ₁ -Akₐ₁,...and/or Ag_{b1}-Ak_{b1},...) absorbed in the wells of the microtiter plate and/or on the solid supports are incubated with a mixture of labelled antigen enzymes (Agₐ₁E*^{,}...Ag_{b1}E*,...) complementary to the antibodies (Akₐ₁, Akₐ₂,...Ak_{b1}, Ak_{b2},...) to be detected, whereupon, after incubation with the mixture of labelled antigen enzymes, the solid supports are inserted into a second microtiter plate, and the activities of the enzymes bound to the complexes (Agₐ₁ -Akₐ₁ -Agₐ₁E*,...Ag_{b1} -Ak_{b1} -Ag_{b1}E*,...) are photometrically determined upon reaction with a staining substrate.

3. A method of simultaneously determining at least one antibody (Akₐ₁,...) and at least one antigen (Ag_{b1},...) in human body liquids by the formation of antibody/antigen or antigen-antibody-complexes, wherein the sample to be assayed for its antibody or antigen content is contacted with a complexing antigen or antibody adsorbed on a solid support, characterised in that the sample is introduced into a microtiter plate and that a plurality of solid supports, preferably in the form of a comb, is contacted with the sample, wherein the wells of the microtiter plate and the solid supports are coated with the antigen(s) (Ag_{b1},...) or antibody (antibodies) (Ak_{b1},...) complementary to the antibody (antibodies) (Akₐ₁,...) or antigen(s) (Ag_{b1},...) to be detected; whereupon - after complexing has been effected - the wells and the solid supports are washed free of non-adsorbed proteins, the complexes (Agₐ₁-Akₐ₁,... or Ak_{b1} -Ag_{b1},...) adsorbed in the wells of the microtiterplate and/or on the solid supports are incubated with a mixture of labelled antigen enzymes (Agₐ₁E*,...) or antibody enzymes (Ak_{b1}E*) complementary to the antibody (antibodies) (Akₐ₁,...) or antigen(s) (Ag_{b1},...) to be detected, the solid supports, after incubation with the mixture of labelled antigen or antibody enzymes, are inserted into a second microtiter plate, and the activities of the enzymes bound to the complexes (Agₐ₁-Akₐ₁ -Agₐ₁E*... or Ak_{b1} -Ag_{b1} -Ak_{b1}E*...) are photometrically determined after reaction with a staining substrate.

4. A kit for carrying out the method according to one or several of claims 1 to 3, with a microtiter plate and a solid support fitting into the wells of the microtiter plate, in particular a comb-like support, characterised in that the wells of the microtiter plate and the solid supports are coated with one or several antigen(s) (Agₐ₁, Ag_{b1},...) or with one or several antibody (antibodies) (Akₐ₁, Ak_{b1},...) which form complexes with the antigen(s) and/or antibody (antibodies) to be detected.

## Revendications

1. Procédé de détermination d'antigènes dans des liquides biologiques humains par formation d'un complexe antigène/anticorps, l'échantillon dont on entend doser la teneur en antigènes étant mis en contact avec un anticorps complexant adsorbé sur un support à l'état solide, caractérisé en ce que pour déterminer simultanément une multiplicité d'antigènes (Agₐ₁, Agₐ₂,... Ag_{b1}, Ag_{b2},...) contenus dans l'échantillon à examiner, on introduit l'échantillon dans une plaque de microtitrage dont les godets sont enduits d'anticorps (Akₐ₁, Akₐ₂,...) dirigés contre une partie des antigènes (Agₐ₁, Agₐ₂,...) recherchés et qu'on met une multiplicité de supports solides, ayant de préférence la forme d'un peigne, enduits d'anticorps (Ak_{b1}, Ak_{b2},...) dirigés contre une autre partie des antigènes (Ag_{b1}, Ag_{b2},...) recherchés, en contact avec l'échantillon; qu'on débarrasse ensuite, par lavage, après réalisation de la complexation, les godets et les supports solides des protéines non adsorbées, qu'on incube les complexes (Akₐ₁-Agₐ₁,... et/ou Ak_{b1}-Ag_{b1},...) adsorbés dans les godets de la plaque de microtitrage et/ou sur les supports solides avec un mélange d'enzymes anticorps marquées (Akₐ₁E*,... Ak_{b1}E*,...) dirigées contre les antigènes (Agₐ₁, Agₐ₂,... Ag_{b1}, Ag_{b2},...) recherchés, qu'on introduit les supports solides après l'incubation avec le mélange d'enzymes anticorps marquées dans une seconde plaque de microtitrage et qu'on détermine l'activité des enzymes liées aux complexes (Akₐ₁-Agₐ₁-Akₐ₁E*,... Ak_{b1}-Ag_{b1}-Ak_{b1}E*,...) par photométrie, après mise à réagir avec un substrat colorant.

2. Procédé de détermination d'anticorps dans des liquides biologiques humains par formation d'un complexe anticorps/antigène, l'échantillon dont on entend doser la teneur en anticorps étant mis en contact avec un antigène complexant adsorbé sur un support à l'état solide, caractérisé en ce que pour déterminer simultanément une multiplicité d'anticorps (Akₐ₁, Akₐ₂,..., Ak_{b1}, Ak_{b2},...) contenus dans l'échantillon à examiner, on introduit l'échantillon dans une plaque de microtitrage dont les godets sont enduits d'antigènes (Agₐ₁, Agₐ₂,...) correspondant à une partie des anticorps (Akₐ₁, Akₐ₂,...) recherchés et qu'on met en contact une multiplicité de supports solides ayant de préférence la forme d'un peigne, enduits d'antigènes (Ag_{b1}, Ag_{b2},...) correspondant à une autre partie des anticorps (Ak_{b1}, Ak_{b2},...) recherchés; qu'on débarrasse ensuite par lavage, après réalisation de la complexation, les godets et les supports solides des protéines non adsorbées, qu'on incube les complexes (Agₐ₁, Akₐ₁,... et/ou Ag_{b1}, Ak_{b1},...) adsorbés dans les godets de la plaque de microtitrage et/ou sur les supports solides avec un mélange d'enzymes antigènes marquées (Agₐ₁E*,... Ag_{b1}E*,...) correspondant aux anticorps (Akₐ₁, Akₐ₂... Ak_{b1}, Ak_{b2},...) recherchés, qu'on introduit les supports solides après l'incubation avec le mélange d'enzymes antigènes marquées dans une seconde plaque de microtitrage et qu'on détermine l'activité des enzymes liées aux complexes (Agₐ₁-Akₐ₁-Agₐ₁E*,... Ag_{b1}-Ak_{b1}-Ag_{b1}E*,...) par photométrie, après mise à réagir avec un substrat colorant.

3. Procédé de détermination simultanée d'au moins un anticorps (Akₐ₁,...) et au moins un antigène (Ag_{b1},...) dans des liquides biologiques humains par formation de complexes anticorps/antigène ou antigène-anticorps, l'échantillon dont on entend doser la teneur en anticorps ou en antigène étant mis en contact avec un antigène ou un anticorps complexant adsorbé sur un support à l'état solide, caractérisé en ce que l'échantillon est introduit dans une plaque de microtitrage et qu'on met une multiplicité de supports solides, ayant de préférence la forme d'un peigne, en contact avec l'échantillon, les godets de la plaque de microtitrage et les supports solides étant enduits de l'(des) antigène(s) (Agₐ₁,...) ou anticorps (Ak_{b1},...) correspondant à l'(aux) anticorps (Akₐ₁,...) ou antigène(s) (Ag_{b1},...) recherché(s); qu'on débarrasse ensuite par lavage, après réalisation de la complexation, les godets et les supports solides des protéines non adsorbées, qu'on incube les complexes (Agₐ₁-Akₐ₁,... ou Ak_{b1}-Ag_{b1},...) adsorbés dans les godets de la plaque de microtitrage et/ou sur les supports solides avec un mélange d'enzymes antigènes (Agₐ₁E*,...) ou d'enzymes anticorps (Ak_{b1}E*,...) marquées correspondant à l'(aux) anticorps (Akₐ₁,...) ou antigène(s) (Ag_{b1},...) recherché(s), qu'on introduit les supports solides après l'incubation avec le mélange d'enzymes antigènes ou enzymes anticorps marquées dans une seconde plaque de microtitrage et qu'on détermine l'activité des enzymes liées aux complexes (Agₐ₁-Akₐ₁-Agₐ₁E*,... ou Ak_{b1}-Ag_{b1}-Ak_{b1}E*,...) par photométrie, après mise à réagir avec un substrat colorant.

4. Kit de mise en oeuvre du procédé selon une ou plusieurs des revendications 1 à 3, comportant une plaque de microtitrage et un support solide adapté aux godets de la plaque de microtitrage, notamment un support de type peigne, caractérisé en ce que les godets de la plaque de microtitrage et les supports solides sont enduits d'un ou plusieurs antigène(s) (Agₐ₁, Ag_{b1},...) ou d'un ou plusieurs anticorps (Akₐ₁, Ak_{b1},...) qui forment des complexes avec l'(les) antigène(s) et/ou anticorps recherché(s).
